(19) Europäisches Patentamt
European Patent Office
Office européen des brevets

(11) Veröffentlichungsnummer: **0 338 429 B1**

(12) **EUROPÄISCHE PATENTSCHRIFT**

(45) Veröffentlichungstag der Patentschrift: **20.01.93**

(51) Int. Cl.[5]: **C07D 311/72**

(21) Anmeldenummer: **89106601.1**

(22) Anmeldetag: **13.04.89**

# (54) Verfahren zur Herstellung von d-alpha-Tocopherol aus natürlichen Vorprodukten.

(30) Priorität: **22.04.88 DE 3813624**

(43) Veröffentlichungstag der Anmeldung:
**25.10.89 Patentblatt 89/43**

(45) Bekanntmachung des Hinweises auf die Patenterteilung:
**20.01.93 Patentblatt 93/03**

(84) Benannte Vertragsstaaten:
**BE CH DE FR GB IT LI NL**

(56) Entgegenhaltungen:
**US-A- 3 819 657**

(73) Patentinhaber: **BASF Aktiengesellschaft**
**Carl-Bosch-Strasse 38**
**W-6700 Ludwigshafen(DE)**

(72) Erfinder: **Lechtken, Peter, Dr.**
**Ludwigshafener Strasse 6B**
**W-6710 Frankenthal(DE)**
Erfinder: **Hoercher, Ulrich, Dr.**
**Kolpingstrasse 15**
**W-6800 Mannheim 1(DE)**
Erfinder: **Jessel, Barbara, Dr.**
**Hochfeldstrasse 26**
**W-6700 Ludwigshafen(DE)**

EP 0 338 429 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von d-α-Tocopherol aus einem aus natürlichen Vorprodukten gewonnenen, α-, β-, γ- und δ-Tocopherole enthaltenden Gemisch durch Methylieren der Tocopherole am aromatischen Ring durch Umsetzen mit Formaldehyd und katalytische Hydrierung.

In der Natur vorkommendes Vitamin E ist ein Gemisch aus α-, β-, γ- und δ-Tocopherol, das stereochemisch einheitlich alle chiralen Zentren in der R-Form aufweist. α,β,γ- und δ-Form unterscheiden sich lediglich im Methylierungsgrad am Benzolring:

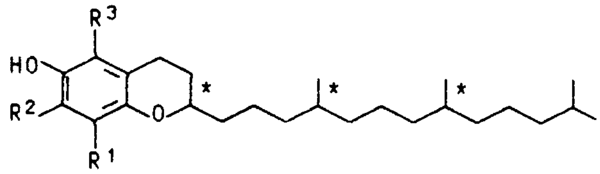

*,*,*=R,R,R (abgekürzt d)

| $R^1$ | $R^2$ | $R^3$ | |
|---|---|---|---|
| $CH_3$ | $CH_3$ | $CH_3$ | d-α-Tocopherol |
| $CH_3$ | H | $CH_3$ | d-β-Tocopherol |
| $CH_3$ | $CH_3$ | H | d-γ-Tocopherol |
| $CH_3$ | H | H | d-δ-Tocopherol |

Die verschiedenen Tocopherole haben unterschiedliche Vitamin-E-Aktivität. Die bei weitem höchste Aktivität besitzt das d-α-Tocopherol und es ist somit das wichtigste Produkt.

Synthetische Methoden zur Herstellung des optisch reinen d-α-Tocopherols haben sich bislang als unwirtschaftlich erwiesen. Deshalb ist man auf die Isolierung dieses Stoffes aus natürlichen Materialien angewiesen. Hierbei kommen insbesondere pflanzliche Fette und Öle, die reich an Tocopherolen sind, wie Soja-, Sonnenblumen-, Raps-, Palm-, Baumwollsaat-, Lein-, Kokos-und Weizenkeimöle, als natürliche Vorprodukte in Betracht.

Besonders bevorzugt ist hierbei das sogenannte Dämpferkondensat, das bei der Desodorierung von Speiseölen mit Wasserdampf erhalten wird, und das 4-15 % Tocopherole enthält.

Da diese natürlichen Tocopherole meist nur einen geringen Anteil an dem hochwirksamen d-α-Tocopherol enthalten, zum überwiegenden Teil aber aus d-β-, d-γ-, und d-δ-Tocopherolen (d.h. nicht -α-Tocopherolen) bestehen, ist es Aufgabe der Erfindung, ein Verfahren zu entwickeln, das kommerziell akzeptabel durchführbar ist, möglichst hochgiftige Chemikalien vermeidet und in hoher Ausbeute zum stereochemisch einheitlichen d-α-Tocopherolführt.

Es sind bereits einige Verfahren zur Umwandlung von nicht -α-Tocoperolen in α-Tocopherol beschrieben worden. Beispielsweise sei die Chlormethylierung mit Formaldehyd und HCl und nachfolgende Reduktion der Chlormethylgruppe zur Methylgruppe mittels $SnCl_2$, metallisches Zink oder Wasserstoff in Gegenwart von Ni oder Pd (vgl. US 2.486.539, US 2.486.542 bzw. US 2.843.604) genannt. Bei diesen Methoden jedoch besteht die Gefahr der Bildung des kanzerogenen Chlordimethylethers. Ein weiterer Nachteil dieser Verfahren ist die Belastung des Abwassers durch Schwermetalle.

Weiterhin ist die Umwandlung von nicht-α-Tocopherolen mit Formaldehyd in Gegenwart von Säuren, wie Orthophosphorsäure (vgl. DE 2 351 272), stark saurer Ionentauscher (vgl. US 4 239 691 oder EP-A 178 400) oder Essigsäure (vgl. JA-OS 56-68677) und anschließende Hydrierung bekannt.

Diese Verfahren sind jedoch auch nicht voll befriedigend, da durch die bei der Hydroxymethylierung verwendeten Säuren eine teilweise Racemisierung am Chiralitätszentrum im Chromanring eintritt, und damit die Aufgabe, nur das in der Natur vorkommende, optisch einheitliche R,R,R-Tocopherol herzustellen, nur unvollkommen gelöst wird.

Zudem bringen die starken Säuren Korrosionsprobleme und damit Verunreinigungen mit Schwermetallen im Tocopherol mit sich, die nur durch Verwendung sehr teurer Spezialstähle als Anlagenmaterial gelöst werden können.

Es war daher die Aufgabe der Erfindung, das an sich vorteilhafte Verfahren zur Umwandlung von nicht-α-Tocopherolen in α-Tocopherole durch Hydroxymethylierung mit Formaldehyd und anschließende katalytische Hydrierung so zu verbessern, daß die Racemisierung am Chiralitätszentrum des Chromanrings möglichst stark vermindert wird und Korrosionsprobleme auch bei Verwendung üblicher Stähle nicht in nennenswertem Umfang auftreten.

Es wurde nun überraschenderweise gefunden, daß d-α-Tocopherol in hohen Ausbeuten aus natürlichen Gemischen von α,β,γ und δ-Tocopherolen herstellbar ist, wenn man die Hydroxymethylierung am Benzolring der nicht-α-Tocopherole durch Formaldehyd in Gegenwart eines Esters der Phosphorsäure oder der phosphorigen Säure vornimmt.

Gegenstand der Erfindung ist dementsprechend ein Verfahren zur Herstellung von d-α-Tocopherol aus einem aus natürlichen Vorprodukten gewonnenen, α,β,γ- und δ-Tocopherole enthaltendem Gemisch durch Methylierungen am aromatischen Ring der Tocopherole durch Umsetzen mit Formaldehyd oder einer unter den Reaktionsbedingungen Formaldehyd abspaltenden Verbindung und anschließende katalytische Hydrierung, das dadurch gekennzeichnet ist, daß man die Umsetzung mit Formaldehyd oder der Formaldehyd abspaltenden Verbindung bei 150 bis 200 °C in Gegenwart eines Alkylesters der Phosphorsäure oder der phosphorigen Säure durchführt.

Während für die Umsetzung von Formaldehyd mit Phenolen eine Reihe von stark sauren oder stark alkalischen Katalysatoren bekannt ist (s. z.B. "Phenolic Resins" by D.F. Gould, Reinhold Plastics Application Series, Reinhold Publishing Corporation, USA 1959, Seite 26 sowie Tabelle S. 28), ist es im erfindungsgemäßen Verfahren erstmals gelungen einen neutralen Katalysator, nämlich einen Ester der Phosphorsäure oder der phosphorigen Säure für die Hydroxymethylierung mit Formaldehyd zu verwenden.

Dieses Verfahren liefert d-α-Tocopherol in Ausbeuten um 90 %, mit vernachlässigbarer Racemisierung am C-2 (≦2 %) und guter Werkstoffbeständigkeit.

Als Ester der phosphorigen oder der Phosphorsäure kommen handelsübliche Alkylester, insbesondere solche, die sich von geradkettigen, verzweigten oder Iso-alkoholen mit ein bis sechs C-Atomen ableiten, in Betracht, wobei diese Alkylreste gleich oder verschieden sein können. Genannt sei insbesondere Trimethylphosphat und Triethylphosphat.

Die anzuwendende Menge an Ester der phosphorigen oder der Phosphorsäure liegt zwischen 5 und 200 %, bezogen auf eingesetztes Tocopherolgemisch, wobei eine Menge von 10 bis 50 % besonders bevorzugt wird.

Den Formaldehyd kann man als gasförmigen Formaldehyd oder in Form seiner wäßrigen Lösung (Formalin) einsetzen oder aber vorzugsweise in Form einer Formaldehyd abspaltenden Verbindung, wie seinen wasserfreien Oligomeren oder Polymeren wie Trioxan, Metaldehyd und Paraformaldehyd.

Mit besonderem Vorteil wird die Umsetzung mit Formaldehyd oder der Formaldehyd abspaltenden Verbindung in einem $C_1$- bis $C_3$-Alkanol oder aber auch in Essigsäuremethylester oder Essigsäureethylester durchgeführt.

Besonders vorteilhaft gestaltet sich die erfindungsgemäße Umwandlung der nicht-α-Tocopherole in α-Tocopherol wenn man die Umsetzung mit Formaldehyd und die katalytische Hydrierung gemeinsam - mehr oder weniger gleichzeitig - in einem Druckgefäß durchführt.
Dabei haben sich Temperaturen zwischen 150 °C und 220 °C besonders bewährt. Die Hydrierung gelingt schon bei niedrigen Wasserstoffdrucken. Im allgemeinen arbeitet man bei Wasserstoffdrucken von 20 bis 150 bar, vorzugsweise 30 bis 100 bar.

Wird die Hydrierung getrennt von der Hydroxymethylierung durchgeführt, gelingt sie schon bei Raumtemperatur.

Als Hydrierkatalysator verwendet man im allgemeinen die üblichen Edelmetallkatalysatoren, wie Palladium und Platin in reiner Form, als Trägerkatalysator oder als Verbindung, wie $PtO_2$. Besonders bewährt hat sich für die erfindungsgemäße Umsetzung Pd auf Aktivkohle mit 5 bis 10 % Pd-Anteil. Aber auch andere Metalle, wie Nickel und Raney-Nickel können verwendet werden.

Als Lösungsmittel werden mit Vorteil $C_1$- bis $C_3$-Alkanole oder aber Essigsäuremethylester oder Essigsäureethylester verwendet.

Eine bevorzugte Ausführungsform des erfindungsgemäßen Verfahrens ist eine quasi-kontinuierliche Arbeitsweise, bei der der Hydrierkatalysator im Druckgefäß vorgelegt wird und die Reaktionskomponenten bei der Reaktionstemperatur unter Wasserstoffdruck im Reaktionsgefäß zusammengeführt werden. Nach der Reaktion wird bei Reaktionstemperatur oder bei einer etwas niedrigeren Temperatur das Reaktionsgefäß über eine Fritte entleert. Der Katalysator verbleibt im Autoklaven und kann nach Spülen mit Methanol erneut eingesetzt werden.

Mit Hilfe des erfindungsgemäßen Verfahrens gelingt die Herstellung von d-α-Tocopherol aus einem aus natürlichen Vorprodukten gewonnenen, α-, β-, γ- und δ-Tocopherole enthaltenden Gemisch durch Methylieren am aromatischen Ring der Tocopherole durch Umsetzen mit Formaldehyd und katalytische Hydrierung

auch bei Verwendung der neutralen Katalysatoren in guten Ausbeuten, wodurch Racemisierungen und Korrosionsprobleme vermieden werden können.

Die folgenden Beispiele illustrieren die erfindungsgemäße Arbeitsweise, ohne limitierend zu sein.

Beispiel 1

80 g eines aus Dämpferkondensat erhaltenen natürlichen Gemisches von Tocopherolen enthaltend 4,0 % $\alpha$-, 1,3 % $\beta$-, 54,8 % $\gamma$- und 40,6 % $\delta$-Tocopherol, wurden zusammen mit 240 g Methanol, 1,12 g 10 % Pd/C, 29,8 g Paraformaldehyd und 25,2 g Trimethylphosphat in einen 1,2 l Autoklaven gegeben. Nach Verschließen wurde der Autoklav 2 x mit $N_2$ gespült, dann 30 bar Wasserstoff aufgepreßt und innerhalb von 1 Stunde (h) auf 200 °C aufgeheizt. Es stellte sich ein Druck von ca. 80 bar ein. Nach 5 h wurde abgekühlt, der Autoklaveninhalt mit n-Hexan herausgespült und der Katalysator abfiltriert. Die Hexan-Phase wurde dreimal mit 250 ml $H_2O$ ausgeschüttelt, die Wasserphase einmal mit Hexan gewaschen. Die Methanol-Phase wurde mit ebensoviel Wasser versetzt, die sich bildende Ölphase abgenommen und zusammen mit der Hexan-Phase am Vakuum-Rotationsverdampfer bei 0,3 mm und 60 °C eingeengt. Man erhielt 71,5 g eines bräunlichen Öls, das zu 95,3 % aus $\alpha$-Tocopherol und nur noch zu 0,3 % aus $\beta$-, 1,15 % aus $\gamma$- und 0,15 % aus $\delta$-Tocopherol bestand.

Die gaschromatographische Analyse (vgl. Noal Cohen et al, Helv. Chim. Acta 64 (4) (1981) S. 1158-1173) zeigte weiterhin, daß das erhaltene $\alpha$-Tocopherol zu 98,4 % aus RRR- und nur zu 1,6 % aus SRR-$\alpha$-Tocopherol bestand.

Beispiel 2 (Vergleichsbeispiel)

Analog zu Beispiel 1 wurden 80 g des beschriebenen natürlichen Tocopherol-Gemisches aus $\alpha$, $\beta$, $\gamma$ und $\delta$-Tocopherol zusammen mit 240 g Methanol, 29,8 g Paraformaldehed, 1,12 g 10 % Pd/C und 12,8 g $P_2O_5$ in einen 1,2 l Autoklaven gegeben. Nach Spülen mit $N_2$ wurden 30 bar Wasserstoff kalt aufgepreßt und innerhalb von 1 h auf 200 °C aufgeheizt. Nach 5 h wurde abgekühlt, der Autoklaveninhalt mit n-Hexan herausgespült, vom Katalysator abgesaugt und der Austrag auf analog Beispiel 1 aufgearbeitet.

Man erhielt 78,4 g eines bräunlichen Öls, das zu 92,3 % aus $\alpha$-Tocopherol und zu 0,57 % aus $\beta$-, 2,63 % aus $\gamma$-und 0,21 % aus $\delta$-Tocopherol bestand.

Die gaschromatographische Analyse zeigte, daß das $\alpha$-Tocopherol zu 94,1 % aus RRR- und zu 5,9 % aus SRR-$\alpha$-Tocopherol bestand.

Dieses Vergleichsbeispiel zeigt, daß die Racemisierung bei Verwendung stark saurer Katalysatoren wesentlich stärker ausgeprägt ist als bei der erfindungsgemäßen Umsetzung in Gegenwart der neutralen Phosphorsäureester (vgl. Beispiel 1).

Beispiel 3

(Vergleich der erfindungsgemäßen Umsetzung mittels neutralem Katalysator mit der bekannten Umsetzung an dem stark sauren Katalysator $P_2O_5$).

In zwei Miniautoklaven (M1 und M2) mit einem Inhalt von 300 ml wurden jeweils 11,3 g eines natürlichen Tocopherol-Gemisches aus Sojaöl, enthaltend 5,68 % $\alpha$-, 1,31 % $\beta$-, 42,75 % $\gamma$- und 38,87 % $\delta$-Tocopherol, zusammen mit 10 g Trioxan, 60 g Methanol und 0,56 g 10 % Pd/C eingefüllt.

Miniautoklav M1 wurde mit 6,3 g Triethylphosphat, Miniautoklav M2 dagegen mit 3,2 g $P_2O_5$ als Katalysator beschickt.

Nach dem Verschließen und Spülen mit $N_2$ wurden jeweils 50 bar Wasserstoff aufgepreßt, unter Rühren (Rührerdrehzahl ca. 800 U/min) auf 200 °C aufgeheizt und bei dieser Temperatur 5 h lang gehalten.

Danach ließ man abkühlen, nahm den Inhalt in Ethanol auf und bestimmte das gebildete Tocopherolgemisch mittels HPLC.

Die erzielten Umsetzungen für sechs aufeinander folgende Versuche sind aus Tabelle 1 ersichtlich. Es zeigte sich, daß Triethylphosphat zu etwa gleichen Ausbeuten und Umsatzgraden führt wie $P_2O_5$.

Tabelle 1

| | Autoklav | Tocopherole im Austrag [%] Alpha | Beta | Gamma | Delta |
|---|---|---|---|---|---|
| a | M 1 | 98,18 | - | - | - |
| | M 2 | 99,41 | - | - | - |
| b | M 1 | 98,48 | - | - | - |
| | M 2 | 99,35 | - | - | - |
| c | M 1 | 96,66 | 0,38 | - | - |
| | M 2 | 92,32 | 4,07 | - | - |
| d | M 1 | 99,54 | - | - | - |
| | M 2 | 99,71 | - | - | - |
| e | M 1 | 95,81 | 3,62 | - | - |
| | M 2 | 100,0 | - | - | - |
| f | M 1 | 100,0 | - | - | - |
| | M 2 | 94,39 | 5,55 | - | - |

Durchschnittlicher Umwandlungsgrad in α-Tocopherol unter Verwendung von Triethylphosphat (M 1) 98,3 % und unter Verwendung von $P_2O_5$ (M 2) 97,5 %.

Beispiel 4

Bei Reaktionsansätzen analog Beispiel 3 wurden in die Autoklaven zusätzlich Material-Prüfplättchen üblicher Stahlsorten mit eingebracht, um die Korrosivität der Materialien zu überprüfen. Die Prüfdauer betrug jeweils ca. 60 - 70 h.

Man erhielt folgende Korrosionsgeschwindigkeiten in mm/a:

| | Stahl (DIN-Nr.) | Triethylphoshat als Katalysator | $P_2O_5$ als Katalysator |
|---|---|---|---|
| a | 1,4571 | 0,3 | 1,51 |
| b | 1,4539 | 0,03 | 1,27 |
| c | 1,4439 | 0,05 | 1,39 |
| d | 1,4465 | 0,08 | 0,55 |

Dieses Beispiel zeigt deutlich, daß die geprüften Stähle gegen das Triethylphosphat enthaltende Reaktionsgemisch noch ausreichend stabil waren, während die Beständigkeit gegen das $P_2O_5$-enthaltene Reaktiosgemisch nicht mehr gegeben ist.

**Patentansprüche**

1. Verfahren zur Herstellung von d-α-Tocopherol aus einem aus natürlichen Vorprodukten gewonnenen, α-, β-, γ- und δ-Tocopherole enthaltendem Gemisch durch Methylieren am aromatischen Ring der Tocopherole durch Umsetzen mit Formaldehyd oder einer unter den Reaktionsbedingungen Formaldehyd abspaltenden Verbindung und anschließende katalytische Hydrierung, dadurch gekennzeichnet, daß man die Umsetzung mit Formaldehyd oder der Formaldehyd abspaltenden Verbindung bei 150 bis

200°C in Gegenwart eines Alkylesters der Phosphorsäure oder der phosphorigen Säure durchführt.

2. Verfahren gemäß Anspruch 1, dadurch gekennzeichnet, daß man die Umsetzung mit Formaldehyd oder der Formaldehyd abspaltenden Verbindung in einem $C_1$- bis $C_3$-Alkanol, Essigsäuremethylester oder Essigsäureethylester durchführt.

3. Verfahren gemäß Anspruch 1, dadurch gekennzeichnet, daß man als Formaldehyd abspaltende Verbindung Paraformaldehyd, Trioxan oder Methylal einsetzt.

4. Verfahren gemäß Anspruch 1 bis 3, dadurch gekennzeichnet, daß man die Umsetzung mit Formaldehyd und die katalytische Hydrierung gemeinsam in einem Druckgefäß durchführt.

5. Verfahren gemäß Anspruch 4, dadurch gekennzeichnet, daß man in einem Druckbereich von 20 bis 150 bar arbeitet.

6. Verfahren gemäß Anspruch 4, dadurch gekennzeichnet, daß man als Hydrierkatalysator einen Edelmetallkatalysator anwendet.

7. Verfahren gemäß Anspruch 1, dadurch gekennzeichnet, daß man die Reaktionskomponenten bei der Reaktionstemperatur unter Wasserstoffdruck im Druckgefäß zusammenführt.

**Claims**

1. A process for the preparation of d-α-tocopherol from a mixture obtained from natural intermediates and containing α-, β, - and δ-tocopherols, by methylation at the aromatic ring of the tocopherols by reaction with formaldehyde or with a compound which donates formaldehyde under the reaction conditions, and subsequent catalytic hydrogenation, wherein the reaction with formaldehyde or with the formaldehyde donor is carried out at from 150 to 200°C in the presence of an alkyl ester of phosphoric or phosphorous acid.

2. A process as claimed in claim 1, wherein the reaction with formaldehyde or with the formaldehyde donor is carried out in a $C_1$-$C_3$-alkanol, methyl acetate or ethyl acetate.

3. A process as claimed in claim 1, wherein the formaldehyde donor used is paraformaldehyde, trioxane or methylal.

4. A process as claimed in any of claims 1 to 3, wherein the reaction with formaldehyde and the catalytic hydrogenation are carried out together in a pressure vessel.

5. A process as claimed in claim 4, wherein a pressure of from 20 to 150 bar is employed.

6. A process as claimed in claim 4, wherein the hydrogenation catalyst used is a noble metal catalyst.

7. A process as claimed in claim 1, wherein the reactants are brought together in the pressure vessel at the reaction temperature under hydrogen pressure.

**Revendications**

1. Procédé de préparation de d-α-tocophérol à partir d'un mélange contenant les α, β, γ et δ-tocophérols obtenu à partir des précurseurs naturels, par méthylation sur le noyau aromatique des tocophérols par réaction avec du formaldéhyde ou avec un composé libérant du formaldéhyde dans les conditions de la réaction, et hydrogénation catalytique ultérieure, caractérisé en ce qu'on effectue la réaction avec le formaldéhyde ou le composé libérant du formaldéhyde à une température de 150 à 200°C en présence d'un ester alkylique de l'acide phosphorique ou de l'acide phosphoreux.

2. Procédé selon la revendication 1, caractérisé en ce qu'on effectue la réaction avec le formaldéhyde ou le composé libérant du formaldéhyde dans un alcanol en $C_1$ à $C_3$ de l'acétate de méthyle ou de l'acétate d'éthyle.

3. Procédé selon la revendication 1, caractérisé en ce qu'on utilise comme compose libérant du formaldéhyde le paraformaldéhyde le trioxanne ou le méthylal.

4. Procédé selon les revendications 1 à 3, caractérisé en ce qu'on effectue la réaction avec le formaldéhyde et l'hydrogénation catalytique simultanément dans un autoclave.

5. Procédé selon la revendication 4, caractérisé en ce qu'on travaille dans une gamme de pressions de 20 à 150 bar.

6. Procédé selon la revendication 4, caractérisé en ce qu'on utilise un catalyseur de métal noble comme catalyseur d'hydrogénation.

7. Procédé selon la revendication 1, caractérisé en ce qu'on introduit ensemble dans l'autoclave les composants de la réaction à la température de la réaction sous pression d'hydrogène.